# EUROPEAN PATENT APPLICATION

(11) **EP 0 629 610 A2**
(43) Date of publication of application: **21.12.1994**
(21) Application number: 94303348.0
(22) Date of filing: 10.05.1994
(51) Int. Cl.: C07C 269/04

(54) **Process for preparation of iodopropargyl carbamates**

(30) Priority: 14.06.1993 US 76660; 14.06.1993 US 76561
(71) Applicant: ROHM AND HAAS COMPANY, Philadelphia, Pennsylvania 19106-2399 (US)
(72) Inventor: Lange, Barry Clifford, Landsdale, Pennsylvania 19446 (US)
(74) Representative: Smith, Julian Philip Howard

(57) **Abstract**

Process for preparing iodopropargyl carbamate compounds comprising forming N-alkyl(C₁-C₆)propargyl carbamate by
either A) reacting together an alkyl(C₁-C₆)amine, liquid supercritical carbon dioxide, propargyl alcohol, and optionally a catalyst;
or B) reacting a dialkyl(C₁-C₃) carbonate with propargyl alcohol in the presence of a first catalyst under conditions to produce dipropargyl carbonate, and then reacting said dipropargyl carbonate with an alkyl(C₁-C₆)amine, optionally in the presence of a second catalyst;
then reacting said N-alkyl(C₁-C₆)propargyl carbamate with an iodinating agent, is disclosed.

## Description

This invention concerns chemical processes for preparation of iodopropargyl carbamates. POLYPHASE® fungicide is one of the leading paint mildewcides in the market place currently. The active ingredient is N-butyl iodopropargyl carbamate.

US 3,923,870 discloses what is presumed to be the commercial process for preparing N-butyl iodopropargyl carbamate. EP 0014032A discloses reacting an alkynol with an isocyanate and followed by iodination. These two processes use butyl isocyanate which is presumably produced from the reaction of butyl amine with phosgene. Isocyanates are generally toxic and are hazardous materials to handle. A more recent process patent, GB 222 0000A published June 23, 1989, discloses a method which uses as a starting material alkynyl chloroformate which is presumably produced from the reaction from propargyl alcohol and phosgene. Alkynyl chloroformate is also toxic, unstable, and hazardous. Although phosgene is a cheap raw material, it is also a very hazardous material. It is likely that the cost of production of N-butyl iodopropargyl carbamate is greatly influenced by the procedures necessary for the safe handling of phosgene.

EP 539092A also discloses a process for the preparation of N-butyl iodopropargyl carbamate that avoids the use of phosgene and isocyanates.

It is an object of the present invention to provide an improved process for preparation of iodopropargyl carbamates. A further object is to provide a process in which phosgene or butyl isocyanate and other hazardous raw materials used in prior art processes need not be handled, and reduced amounts of solvent may be used. A further object is to provide a process where any unreacted propargyl alcohol can be recycled.

The present invention provides a process for preparing iodopropargyl carbamate compounds, comprising forming N-alkyl(C₁-C₆)propargyl carbamate by
either A) reacting together an alkyl(C₁-C₆)amine, liquid supercritical carbon dioxide, propargyl alcohol, and optionally a catalyst;
or B) reacting a dialkyl(C₁-C₃) carbonate with propargyl alcohol in the presence of a first catalyst under conditions to produce dipropargyl carbonate, and then reacting said dipropargyl carbonate with an alkyl(C₁-C₆)amine, optionally in the presence of a second catalyst;
then reacting said N-alkyl(C₁-C₆)propargyl carbamate with an iodinating agent.

While the process can be used to produce N-alkyl(C₁-C₆) iodopropargyl carbamates, the preferred product is N-butyl iodopropargyl carbamate.

For process route A, the preferred alkyl amine is n-butyl amine. Suitable optional catalysts are dessicants, such as silica gel, molecular sieves, and the like; acids, such as acetic acid and sulfuric acid; metal catalysts; and the like.

The first step in route A comprises reacting an alkyl(C₁-C₆)amine with gaseous or liquid, supercritical carbon dioxide, usually in excess. Typically, one equivalent of amine is reacted with from about 5 to about 50 molar equivalents of carbon dioxide, with 10 molar equivalents being preferred. The mixture is agitated for up to 12 hours. Following this reaction, up to 99% of the original amount of carbon dioxide can be removed. To the reaction mixture is added from about 1 to about 10 molar equivalents of propargyl alcohol, with 2 molar equivalents being preferred. The mixture is then agitated with heating (up to 150°C) and pressurized up from about to 10 to about 50 atm, with 100 atm being preferred. The reaction is completed in from about 0.5 to about 8 hours. The preferred reaction time is 4 hours. Once the reaction is complete, excess propargyl alchol and any unreacted alkyl(C₁-C₆)amine are removed under reduced pressure. The propargyl alcohol and alkyl(C₁-C₆)amine so removed may be recycled. When employed, typical optional dessicants are in the ranges of from about 2 to about 10 grams, with 5 grams being preferred.

Process route B is preferably run in the absence of a solvent. However, if desired a solvent could be used. Suitable solvents are those with boiling points higher than the alcohol released during the first step of the reaction. In the case of dimethyl carbonate, methanol is released; in the case of diethyl carbonate, ethanol is released and propanol is released in the case of dipropyl carbonate. Examples of suitable solvents are propargyl alcohol, aromatic hydrocarbons, glymes, high boiling alkanes, and the like. By aromatic hydrocarbons is meant toluene, xylene, and the like. By glymes is meant diglyme, triglyme, and the like. By high boiling alkanes is meant decalin and the like. Azeotropes of suitable solvents are also contemplated, provided the boiling point is as specified for the solvents above.

The preferred carbonates are dimethyl carbonate and diethyl carbonate.

Suitable first catalysts for route B are any which promote the desired reaction. A number of such catalysts are known in the art. Examples of suitable first catalysts are boron tribromide, trimethylsilyliodide/iodine, potassium cyanide, Al₂O₃, ammonia, n-butyl lithium, alkali metal alkoxide, potassium t-butoxide/molecular sieves, dimethylaminopyridine, Ti(OCH₂CH₃)₄, sulfuric acid, p-toluenesulfonic acid, and the like. Suitable alkali metal alkoxides are sodium or potassium methoxide, sodium or potassium ethoxide, and the like.

As in route A, the preferred alkyl amine is n-butyl amine.

Suitable optional second catalysts for route B are any which promote the desired reaction and may either be acids or bases. Preferred second catalysts are carboxylic acids with acetic acid being most preferred.

The first step in route B comprises reacting a dialkyl(C₁-C₃) carbonate with propargyl alcohol and a first catalyst at reflux to yield dipropargyl carbonate. Suitable ranges of amounts of propargyl alcohol are from about 5 to about 20 molar equivalents based upon dialkylcarbonate, with 10 molar equivalents being preferred. Suitable ranges of amounts of a first catalyst are from about 0.005 to about 0.05 molar equivalents based on dialkylcarbonate. The preferred amount of first catalyst is 0.01 molar equivalents. The reaction is considered complete when the alcohol(C₁-C₃) released during the reaction stops distilling off. To the reaction mixture is added from about 2 to about 10 molar equivalents of a alkyl(C₁-C₆)amine, based on the carbonate produced in the first step. The preferred amount of alkyl(C₁-C₆)amine is 3 molar equivalents. Optionally, a second catalyst may be added. This second step of the reaction is carried out at from 50° to about 115°C until the reaction is complete (1-24 hrs.).

The final process step of iodination of the N-alkyl(C₁-C₆)propargyl carbamate may be performed by a variety of methods and reagents known in the literature. These methods include using iodine or an iodine releaser. Suitable iodine releasers are iodine/amino compounds, such as iodine/morpholine comples, and N-iodosuccinimide. Iodine and N-iodosuccinimide are preferred reagents.

When iodine or an iodine/amino complex is used, base should also be used and solvent such as methanol, ethanol, and aqueous ethanol should be used. Suitable bases include sodium carbonate, potassium carbonate, sodium hydroxide, and potassium hydroxide. Preferred bases are sodium or potassium hydroxide. When N-iodosuccinimide is used, a catalyst, such as silver nitrate or the like, should be used in the presence of a solvent such as acetone, methyl ethyl ketone, tetrahydrofuran, and the like. The preferable method of iodination is to react iodine with N-alkyl(C₁-C₆) propargyl carbamate in the presence of sodium hydroxide. The iodination step is carried out at temperatures of about 0°C to about 25°C for about 20 minutes to about 24 hours.

The following examples illustrate a few embodiments of the invention; however, the invention should not be construed as being limited to these few illustrative embodiments.

### EXAMPLES

### Example 1: Synthesis of N-Butyl Iodopropargyl Carbamate via route A

A. One equivalent of n-butylamine is added to a pressurized vessel charged with 10 molar equivalents of liquid, supercritical carbon dioxide. The mixture is agitated at -30°C at a pressure of 7 atmospheres for 12 hours. Following this, the excess carbon dioxide is removed. To the resulting residue, propargyl alcohol (2 molar equivalents) and dry silica gel (5 grams) is added and the reaction mixture agitated, heated to 150°C and pressurized to 100 atm. The mixture is reacted under these conditions for 4 hours. Once the reaction is complete, excess propargyl alcohol and unreacted n-butylamine are removed under reduced pressure for recycle, yielding an oil. The oil is taken up in ethyl acetate, washed with water, dried over MgSO₄, filtered, and cleaned up with column chromatography (silica gel with ethyl acetate as eluant) to yield a colourless oil.
B. Iodine (3.3 g 0.013 mol) is added in portions to a stirred solution of the N-butyl propargyl carbamate (4.0 g, 0.026 mole), prepared as above, in ethanol (25 ml), water (10 ml) and 50 % sodium hydroxide (2.1g, 0.026 mol) at 0-5°C. At the end of the iodine addition, the mixture is stirred at the same temperature for another 5 min. A commercial bleach (18.3 g, 5.25 %, 0.013 mole) is then added dropwise to the above solution keeping the temperature at 0-5°C. At the end of the bleach addition, the light yellow solution is stirred at the same temperature for one hour. Extraction with methylene chloride (2 x 70 ml) and evaporation of the solvent on a rotary evaporator gives a crystalline residue. Crystallization from hexane/ toluene gives the N-butyl iodopropargyl carbamate as needles.

### Example 2: Synthesis of N-Butyl Iodopropargyl Carbamate via route B

A. A vessel is charged with dimethyl carbonate (10 g) and 10 mole equivalents of propargyl alcohol and 0.01 mole equivalent of sodium methoxide. The reaction mixture is heated at reflux with stirring and nitrogen atmosphere until the methanol has stopped distilling. Excess propargyl alcohol is distilled off. The residue is cooled to ambient temperature. To this cooled material is added n-butylamine (3 molar equivalents). The mixture is heated to 115°C for 8 hours. The residual propargyl alcohol and excess n-butylamine are distilled off under reduced pressure for recycling. The resultant oil is taken up in ethyl acetate, washed with water, dried over MgSO₄, filtered, and cleaned up with column chromatography (silica gel with ethyl acetate as eluant) to yield a colourless oil.
B. Iodine (3.3 g, 0.013 mole) is added in portions to a stirred solution of the N-butyl propargyl carbamate (4.0 g, 0.026 mole) prepared in A, in ethanol (25 ml), water (10 ml) and 50 % sodium hydroxide (2.1 g, 0.026 mole) at 0-5°C. At the end of the iodine addition, the mixture is stirred at the same temperature for another 5 min. A commercial bleach (18.3 g, 5.25 %, 0.013 mole) is then added dropwise to the above solution keeping the temperature at 0-5°C. At the end of the bleach addition, the light yellow solution is stirred at the same temperature for one hour. Extraction with methylene chloride (2 x 70 ml) and evaporation of the solvent on a rotary evaporator gives a crystalline residue. Crystallization from hexane/toluene gives needles of N-butyl iodopropargyl carbamate.

While the invention has been described in sufficient detail for those skilled in the art to be able to make and use it, various alternatives, modifications, and improvements should become apparent from the foregoing disclosure.

## Claims

1. Process for preparing iodopropargyl carbamate compounds, comprising forming N-alkyl(C₁-C₆)propargyl carbamate by
either A) reacting together an alkyl(C₁-C₆)amine, liquid supercritical carbon dioxide, propargyl alcohol, and optionally a catalyst;
or B) reacting a dialkyl(C₁-C₃) carbonate with propargyl alcohol in the presence of a first catalyst under conditions to produce dipropargyl carbonate, and then reacting said dipropargyl carbonate with an alkyl(C₁-C₆)amine, optionally in the presence of a second catalyst;
then reacting said N-alkyl(C₁-C₆)propargyl carbamate with an iodinating agent.

2. Process according to claim 1 wherein said alkyl(C₁-C₆)amine comprises methyl amine, ethyl amine, propyl amine or n-butyl amine, preferably n-butyl amine.

3. Process according claim 1 or 2 wherein said iodinating agent is a mixture of iodine and a base, said base preferably being sodium hydroxide.

4. Process according to claim 1, 2 or 3 wherein in A one equivalent of amine is reacted with from 5 to 50 molar equivalents, preferably at least 10 molar equivalents, of carbon dioxide.

5. Process according to any preceding claim wherein the catalyst in A comprises dessicants, molecular sieves, acids, or metal catalysts; preferably acetic acid or sulfuric acid.

6. Process according to claim 1, 2 or 3 wherein said dialkyl carbonate comprises dimethyl carbonate or diethyl carbonate.

7. Process according to any of claims 1 to 3 or 6 wherein said first catalyst in B comprises boron tribromide, trimethylsilyliodide/iodine, potassium cyanide, Al₂O₃, ammonia, n-butyl lithium, alkali metal alkoxide, potassium t-butoxide/molecular sieves, dimethylaminopyridine, Ti(OCH₂CH₃)₄ or p-toluenesulfonic acid.

8. Process according to claim 7 wherein said first catalyst comprises sodium methoxide, potassium methoxide, sodium ethoxide or potassium ethoxide, preferably sodium methoxide.

9. Process according to any of claims 1 to 3 or 6 to 8 wherein said second catalyst in B comprises acetic acid.
